# EUROPEAN PATENT SPECIFICATION

(11) **EP 3 439 581 B1**
(45) Date of publication and mention of the grant of the patent: **29.04.2020**
(21) Application number: 17713768.4
(22) Date of filing: 03.02.2017
(51) Int. Cl.: A61F 2/00

(54) **NON-INVASIVE DEVICE AGAINST URINARY INCONTINENCE**
NICHTINVASIVE VORRICHTUNG GEGEN HARNINKONTINENZ
DISPOSITIF NON INVASIF CONTRE L'INCONTINENCE URINAIRE

(30) Priority: 05.02.2016 IT UB20160253
(43) Date of publication of application: 13.02.2019
(73) Proprietor: Melo S.r.l., 25124 Brescia (IT)
(72) Inventor: DONATI, Giancarlo, I-24060 Rogno (BG) (IT); FARDELLI, Ernesto, I-24060 Rogno (BG) (IT); SPAGNOLI, Massimo, I-46043 Castiglione delle Stiviere (MN) (IT)
(74) Representative: Borsano, Corrado
(86) International application number: PCT/IB2017/050588
(87) International publication number: WO 2017/134612

(56) References cited:
- WO-A1-99/43276
- GB-A- 2 407 986
- US-A- 5 571 125
- US-A1- 2004 112 392

## Description

### Field of the invention

The present invention relates to a non-invasive device against urinary incontinence.

### Background art

So far, no effective, safe, non-invasive and non-traumatic remedy has been found to the problem of male incontinence.

In fact, adult diapers or sanitary napkins are still the items most commonly used to counter the effects of incontinence. However, it is clear that the use of absorbing materials and/or devices cannot be considered to be a solution to the problem.

Narrowing the research to systems aimed at solving the problem of male incontinence in a non-invasive, totally non-traumatic manner, diapers excluded, numerous devices are known in the art which simply compress the user's penis by exerting additional pressure on the urethral duct. Such pressure, which is aimed at closing the urinary duct, can be obtained by using a special geometry directly pressing against the urethral duct or via a mechanical action exerted by movable or semi-movable parts that can be actuated by screwing or compression.

This direct pressure against the urethral duct necessarily creates a point of unstable equilibrium for the penis inside the device. When the user makes movements that are common in everyday life, such as picking up keys from the ground, getting out of a car or standing up from a chair, such instability translates into a shift of the penis to the right or to the left of the initial point of equilibrium of the device, thereby causing the urinary duct to open and urine to leak out.

If this were not true, there would be no reason to use diapers.

The invasive anti-incontinence devices already available will not be taken into account herein, since they are difficult to use and subject the urinary duct to a strong risk of infection (typically by Escherichia Coli).

Due to the instability shown by the non-invasive devices against male incontinence currently known in the art, such devices cannot be considered to be functional and effective.

US-5571125-A discloses a non-invasive device against urinary incontinence, adapted to be applied to the external part of a human penis, the device comprising a first part adapted to operationally exert an even compression force on the upper part of the penis, and a second part adapted to operationally exert direct compression forces onto a lower part of the penis.

It is therefore one object of the present invention to provide a non-invasive device against urinary incontinence which can overcome all of the above-mentioned problems.

Following studies and practical tests, a device has been defined which, thanks to its own particular geometry, considerably improves the reliability of this type of non-invasive anti-incontinence devices.

The device according to the invention has a geometry that, unlike prior-art solutions, does not exert pressure directly on the urethral duct, but on the cavernous bodies that surround the urethral duct.

This pressure exerted on the cavernous bodies is indirectly transferred to the walls of the urethral duct, which will thus tend to close the urinary duct.

The advantage of the present invention lies in the fact that it ensures stability of the penis in the correct position also during and/or following everyday movements and the like.

Compared to prior-art devices, the innovation brought about by this invention consists of transforming a point of unstable equilibrium (as is typical of prior-art devices) into a point of stable equilibrium, delegating the task of pressing against the urethral duct to the cavernous bodies, thereby preventing urine from leaking out. The urethral duct is thus compressed indirectly via direct pressure exerted on the cavernous bodies.

The present invention relates to a non-invasive device against urinary incontinence, adapted to be applied to the external part of a human penis and so shaped as to operationally encircle the penis by compression, said penis internally comprising a urethral duct in a lower position and two cavernous bodies in lateral positions, the device being characterized in that it comprises a first part adapted to operationally exert an even compression force on the upper part of the penis, and a second part adapted to operationally exert direct compression forces on both cavernous bodies and indirect compression forces on the urethral duct, induced by said direct compression forces exerted on both cavernous bodies, so as to achieve a stable lateral compression for closing the urethral duct.

In particular, the present invention relates to a non-invasive device against urinary incontinence as specifically set out in the claims, which are an integral part of the present description.

### Brief description of the drawings

Further objects and advantages of the present invention will become apparent from the following detailed description of a preferred embodiment (and variants thereof) referring to the annexed drawings, which are only supplied by way of non-limiting example, wherein:
Figure 1 shows a side view of one embodiment of the device according to the invention;
Figure 2 shows one embodiment of the inner part of the device;
Figures 3 and 4 show side views of the device of Figure 1;
Figure 5 is a cross-sectional view of a penis;
Figure 6 is an explanatory graph showing some examples of the curvilinear profile of the lower inner part of the device;
Figure 7 schematically shows how the lines of force developed by the device can compress the penis.

In the drawings, the same reference numerals and letters identify the same items or components.

### Detailed description of some embodiments of the invention

Once worn, the device exerts an even pressure on the upper part of the penis; this pressure is then transferred to the lower part of the penis, pressing against the geometry of the lower part of the device.

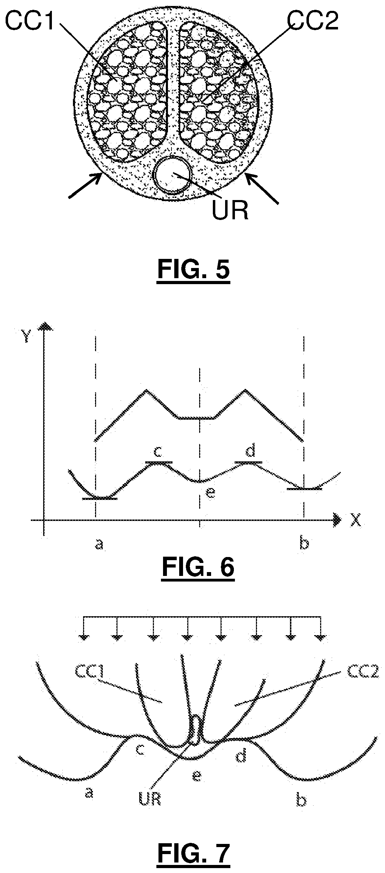

The above-mentioned upper and lower parts of the penis and of the device refer to the shape of the penis cross-section as shown in Fig. 5, which highlights the urethral duct UR at the bottom and two cavernous bodies CC1, CC2 at the sides.

The device 1 is provided in the form of a band encircling the penis in a firm and removable manner, which comprises at least one reclosable opening allowing the device to be arranged in such a way that the penis will remain operationally compressed by it.

The device comprises an upper part 2 and a lower part 3 opposite to each other. Furthermore, in one embodiment the lower and upper parts are fixedly connected together on one side 4, whereas on the other side they are connected by closing means, e.g. two flaps 5', 5" adapted to be overlapped for closing and opening the device around the penis. Closing can be effected by means of Velcro strips or other systems such as soft plastic ties for fishbone or saw-tooth or rack-type fastening.

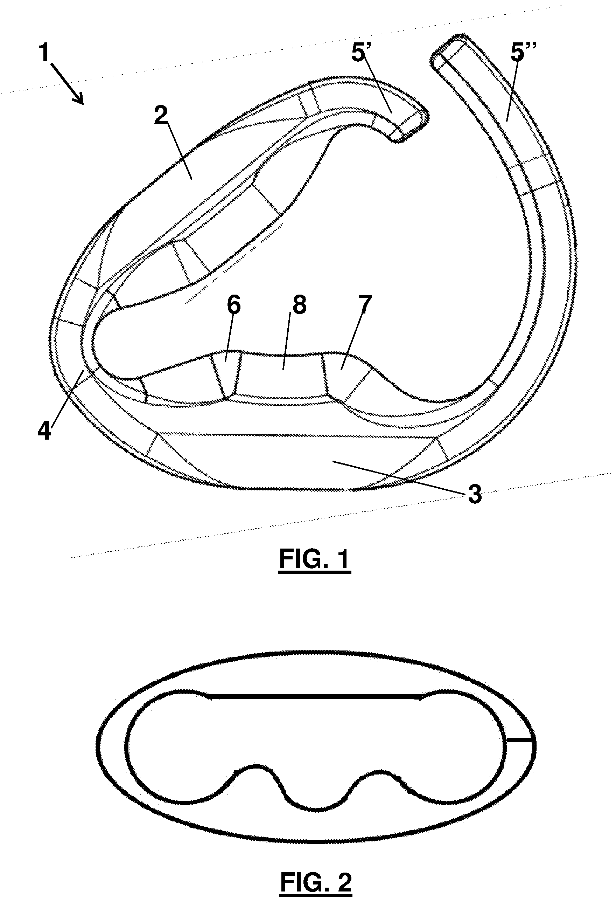

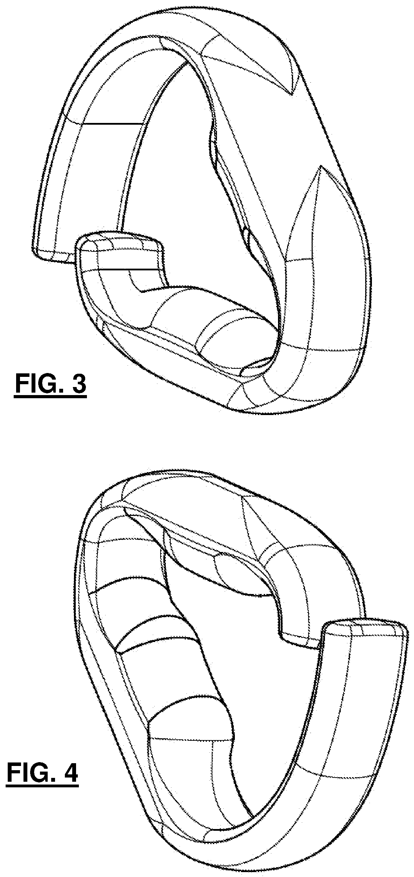

The device preferably comprises a rigid inner layer, e.g. shaped as shown in Fig. 2, coated with a soft foam material part, preferably of antibacterial nature, to adapt itself to the shape of the penis, thus having an overall shape like the one shown by way of example in Figures 1,3,4.

One important aspect of the invention is the geometry of the profile of the lower part 3 of the device. It has a curvilinear shape. As shown in Figures 6 and 7, an area is defined between two lateral points of absolute minimum a and b, between which two points of relative maximum c and d are defined, with a point of relative minimum e in between. This can be achieved by means of curvatures or broken lines of the rigid inner material, the structure being then effectively modelled by the soft external material.

The exemplary curves drawn on the x-y axes, where a < x < b, highlight a curvature having points of horizontal tangency (derivative = 0), or a broken line with inward points of maximum and minimum. As aforesaid, a and b are points of absolute minimum, e is a point or area of relative minimum in a substantially central position relative to c and d, which are points or areas of relative maximum.

Point e is a point of stable equilibrium because it is contoured by c and d points, as opposed to prior-art solutions, wherein the central point is a point of unstable equilibrium directly compressing the urethral duct.

The two lateral protuberances 6, 7 exert a pressure with a lateral force component towards the cavernous bodies (positioned at the sides of the urethral duct), which, being affected by such field of forces orthogonally to the curve surface, close and compress the urethral duct in between. It can be noticed (Figure 7) that the urethral duct UR is compressed by a vector field of forces that is different from the one achievable with prior-art devices: the upper part of the device exerts an even field of forces on the penis, while the lower part, instead of compressing the urethral duct UR directly from below, compresses it laterally and indirectly, i.e. compression is exerted directly and laterally on the cavernous bodies CC1, CC2 by lateral force components, causing the urethral duct to become deformed between the two cavernous bodies.

Such field of forces (or pressures) generated by the specific geometry of this device ensures that the urethral duct will stay properly closed even during movements, since the penis will be at a point of stable equilibrium, unlike current anti-incontinence devices, wherein the initial position is a point of unstable equilibrium.

Therefore, the basic idea is to exert lateral pressure on the cavernous bodies beside the urethral duct, thus creating a point of stable equilibrium to exert pressure on the urethral duct. This provides stability, comfort of use and uninterrupted efficiency.

The rigid inner body (Fig. 2) is made, for example, of thermoplastic material, preferably Nylon 6 (PA 6).

As alternatives to Nylon 6, the following materials may be employed: PP, PE, PA11, PA12, PA66, PA6-66, PC, ABS, PEEK, rigid PVC, plasticized PVC, TPU, or any thermoplastic polymeric material having a sufficient modulus of elasticity (> 40 MPa of tensile modulus) for creating the inner structure, or blends thereof.

Said rigid component represents the inner part of the device.

Said component is manufactured by injection moulding.

Externally to said rigid component, a layer of soft material is applied by overmoulding or coating; the layer of soft material is preferably made of antibacterial thermoplastic foam material.

Such soft, elastic and light coating can also be obtained by dipping, spraying, binding, deposition or the like, and its thickness is in the range of 0.5 to 10 mm depending on the mechanical characteristics of the material used for said coating.

Other materials may also be used for such coating: EVA polymer foam, SEBS foam, SBS foam, TPU foam, PS foam, polyisoprene, latex, silicone and other thermoplastic or thermosetting polymers, or blends thereof.

Open-cell or closed-cell foam materials are to be preferred for this purpose, due to their lower weight as well as their lower environmental impact (and disposal costs).

As previously mentioned, the device includes a fastening system that can be modulated and adapted to each user; for example, closure adjustment can be provided by Velcro strips, but other adjustable closing systems can be adopted as well, whether integral with or separate from the coating geometry.

The above-described non-limiting example of embodiment may be subject to variations without departing from the protection scope of the present invention, including all equivalent designs known to a man skilled in the art.

The elements and features shown in the various preferred embodiments may be combined together without however departing from the protection scope of the present invention. The advantages deriving from the application of the present invention are apparent.

This device has been developed, tested and validated by means of practical tests to ensure the absence of leaks in individuals suffering from male incontinence. Clear benefits to the user's life style and quality are easily imaginable, since the use of diapers is avoided. The numerous advantages also include a smaller environmental impact of this device as concerns the costs and the pollution related to disposal and production of traditional disposable diapers.

In fact, due to its innovative antibacterial component and washable materials, this device can be offered as a reusable, as opposed to disposable, device.

It is important to underline that this solves the problem of the point of unstable equilibrium, which is characteristic of prior-art solutions, and which gives rise to problems, e.g. caused by the person's movements, that can cause the pressure element to shift outside the area of the urethral duct, thus making the device ineffective or even harmful, since it can only be effective in particular conditions, e.g. when the person is still, e.g. sitting in a wheel chair or lying in bed, such effectiveness disappearing if the person moves.

From the above description, those skilled in the art will be able to produce the object of the invention without introducing any further construction details.

## Claims

1. Non-invasive device against urinary incontinence, adapted to be applied to an external part of a human penis and so shaped as to operationally encircle the penis by compression, said penis, in a cross sectional view, internally comprising a urethral duct (UR) in a lower position and two cavernous bodies (CC1, CC2) in lateral positions, the device comprises a first part (2) and a second part (6, 7, 8) arranged on an opposite side of said first part, **characterized in that**
said first part (2) being shaped to operationally exert, in use, an even compression force on the upper part of the penis,
said second part (6, 7, 8) being shaped to operationally exert direct compression forces on both cavernous bodies but only indirect compression forces on the urethral duct, induced by said direct compression forces exerted on both cavernous bodies, so as to achieve a stable lateral compression for closing the urethral duct.

2. Device according to claim 1, wherein said second part (6, 7, 8) comprises a curvilinear inner profile, with two points or areas of relative maximum (c, d), with a point or area of relative minimum (e) in between, said points or areas of relative maximum (c, d) being adapted to exert said direct compression forces on the two cavernous bodies, respectively, said direct compression forces comprising a lateral component.

3. Device according to claim 1, comprising an inner part made of rigid material and an outer part made of soft material, which covers said inner part.

4. Device according to claim 3, wherein said rigid inner part is made of thermoplastic material, or PP, PE, PA11, PA12, PA66, PA6-66, PC, ABS, PEEK, rigid PVC, plasticized PVC, TPU, or blends thereof.

5. Device according to claim 3, wherein said outer part is made by dipping, or spraying, or binding, or deposition, and comprises materials such as EVA polymer foam, SEBS foam, SBS foam, TPU foam, PS foam, polyisoprene, latex, silicone and other thermoplastic or thermosetting polymers, or blends thereof.

6. Device according to any one of the preceding claims, comprising at least one reclosable opening adapted to allow positioning the device in a manner such that the penis will remain operationally compressed.

7. Device according to claim 6, wherein said at least one reclosable opening comprises closing means (5', 5") adapted to be operationally overlapped for closing and opening the device around the penis.

8. Device according to claim 6 or 7, wherein said at least one reclosable opening is implemented by means of Velcro strips, or soft plastic ties for fishbone or saw-tooth or rack-type fastening.

9. Device according to claim 3, wherein, in said inner part, said second part has a curvilinear inner profile comprising bends or broken lines.

## Patentansprüche

1. Nicht-invasive Vorrichtung gegen Harninkontinenz, die zur Anwendung an einem äußeren Teil eines menschlichen Penis geeignet ist und so geformt ist, dass sie den Penis durch Kompression operativ umgibt, wobei der Penis in einer Querschnittsansicht im Inneren einen Harnröhrenkanal (UR) in einer unteren Position und zwei kavernöse Körper (CC1, CC2) in seitlichen Positionen umfasst, wobei die Vorrichtung einen ersten Teil (2) und einen zweiten Teil (6, 7, 8) umfasst, der auf einer gegenüberliegenden Seite des ersten Teils angeordnet ist, **dadurch gekennzeichnet, dass**
das erste Teil (2) derart geformt ist, dass er bei der Anwendung gleichmäßige Druckkraft auf den oberen Teil des Penis operativ ausübt,
wobei der zweite Teil (6, 7, 8) so geformt ist, dass er direkte Kompressionskräfte auf beide kavernösen Körper, aber nur indirekte Kompressionskräfte auf den Harnröhrengang operativ ausübt, die durch die auf beide kavernösen Körper ausgeübten direkten Kompressionskräfte induziert werden, um eine stabile seitliche Kompression zum Verschließen des Harnröhrengangs zu erreichen.

2. Vorrichtung nach Anspruch 1, bei der der zweite Teil (6, 7, 8) ein kurvenförmiges inneres Profil mit zwei Punkten oder Bereichen mit einem relativen Maximum (c, d) und einem Punkt oder Bereich mit einem relativen Minimum (e) dazwischen umfasst, wobei die Punkte oder Bereiche mit einem relativen Maximum (c, d) so angepasst sind, dass sie die direkten Kompressionskräfte auf die beiden kavernösen Körper ausüben, wobei die direkten Kompressionskräfte eine seitliche Komponente umfassen.

3. Vorrichtung nach Anspruch 1, bestehend aus einem Innenteil aus starrem Material und einem Außenteil aus weichem Material, das das Innenteil bedeckt.

4. Vorrichtung nach Anspruch 3, wobei das starre Innenteil aus thermoplastischem Material oder aus PP, PE, PA11, PA12, PA66, PA6-66, PC, ABS, PEEK, Hart-PVC, Weich-PVC, TPU oder Mischungen davon hergestellt ist.

5. Vorrichtung nach Anspruch 3, wobei das Außenteil durch Tauchen oder Sprühen oder durch Binden oder Abscheiden hergestellt wird und Materialien wie EVA-Polymerschaum, SEBS-Schaum, SBS-Schaum, TPU-Schaum, PS-Schaum, Polyisopren, Latex, Silikon und andere thermoplastische oder wärmehärtbare Polymere oder Mischungen davon umfasst.

6. Vorrichtung nach einem der vorstehenden Ansprüche, mit mindestens einer wiederverschließbaren Öffnung, die es ermöglicht, die Vorrichtung so zu positionieren, dass der Penis operativ komprimiert bleibt.

7. Vorrichtung nach Anspruch 6, wobei die mindestens eine wiederverschließbare Öffnung Verschlussmittel (5', 5") umfasst, die so angepasst sind, dass sie sich operativ überlappen, um die Vorrichtung um den Penis herum zu schließen und zu öffnen.

8. Vorrichtung nach Anspruch 6 oder 7, wobei die mindestens eine wiederverschließbare Öffnung mittels Klettstreifen oder Weichplastikbändern für Fischgräten- oder Sägezahn- oder Zahnstangenartige Verschlüsse implementiert ist.

9. Vorrichtung nach Anspruch 3, wobei, in dem Innenteil, der zweite Teil ein kurvenförmiges inneres Profil mit Biegungen oder unterbrochenen Linien aufweist.

## Revendications

1. Dispositif non invasif contre l'incontinence urinaire, ledit dispositif étant adapté pour être appliqué sur une partie extérieure d'un pénis humain et conçu de manière à encercler fonctionnellement le pénis par compression, ledit pénis comprenant à l'intérieur, en coupe transversale, un canal urétral (UR) en position basse et deux corps caverneux (CC1, CC2) en positions latérales, le dispositif comprenant une première partie (2) et une deuxième partie (6, 7, 8) disposées sur un côté opposé de ladite première partie, **caractérisé en ce que**
ladite première partie (2) est formée de manière à exercer fonctionnellement, en utilisation, une force de compression uniforme sur la partie supérieure du pénis,
ladite deuxième partie (6, 7, 8) est formée de manière à exercer fonctionnellement des forces de compression directes sur les deux corps caverneux mais uniquement des forces de compression indirectes sur le canal urétral, lesquelles sont induites par lesdites forces de compression directes exercées sur les deux corps caverneux, de manière à obtenir une compression latérale stable permettant de fermer le canal urétral.

2. Dispositif selon la revendication 1, ladite deuxième partie (6, 7, 8) ayant un profil intérieur curviligne pourvu de deux points ou zones de maximum relatif (c, d) entre les lesquels se trouve un point ou une zone de minimum relatif (e), lesdits points ou lesdites zones de maximum relatif (c, d) étant adaptés pour exercer lesdites forces de compression directes sur chacun des deux corps caverneux, lesdites forces de compression directes comprenant une composante latérale.

3. Dispositif selon la revendication 1, comprenant une partie intérieure en matériau rigide et une partie extérieure en matériau souple qui recouvre ladite partie intérieure.

4. Dispositif selon la revendication 3, ladite partie intérieure rigide étant en matière thermoplastique, ou PP, PE, PA11, PA12, PA66, PA6-66, PC, ABS, PEEK, PVC rigide, PVC plastifié, TPU, ou des mélanges de ceux-ci.

5. Dispositif selon la revendication 3, ladite partie extérieure étant réalisée par trempage, ou pulvérisation, ou liaison, ou dépôt, et comprenant des matières tels que de la mousse polymère EVA, de la mousse SEBS, de la mousse SBS, de la mousse TPU, de la mousse PS, du polyisoprène, du latex, du silicone et autres polymères thermoplastiques ou thermodurcissables, ou leurs mélanges.

6. Dispositif selon l'une quelconque des revendications précédentes, comprenant au moins une ouverture refermable adaptée pour permettre le positionnement du dispositif d'une manière telle que le pénis restera fonctionnellement comprimé.

7. Dispositif selon la revendication 6, ladite au moins une ouverture refermable comprenant des moyens de fermeture (5', 5") adaptés pour se chevaucher fonctionnellement pour fermer et ouvrir le dispositif autour du pénis.

8. Dispositif selon la revendication 6 ou 7, ladite au moins une ouverture refermable étant réalisée au moyen de bandes velcro, ou d'attaches en matière plastique souple pour obtenir une fixation en arête de poisson ou en dents de scie ou en crémaillère.

9. Dispositif selon la revendication 3, ladite deuxième partie présente, dans ladite partie intérieure, un profil intérieur curviligne comprenant des courbes ou des lignes brisées.
